# EUROPEAN PATENT SPECIFICATION

(11) **EP 1 516 006 B1**
(45) Date of publication and mention of the grant of the patent: **05.07.2006**
(21) Application number: 03703411.3
(22) Date of filing: 23.01.2003
(51) Int. Cl.: C08G 63/664, C08G 63/66, A61L 29/00, A61L 31/00

(54) **BLOCK COPOLYMERS FOR SURGICAL ARTICLES**
BLOCKCOPOLYMERE FÜR CHIRURGISCHE ARTIKEL
COPOLYMERES BLOCS DESTINES A DES ARTICLES CHIRURGICAUX

(30) Priority: 26.06.2002 KR 2002035834
(43) Date of publication of application: 23.03.2005
(73) Proprietor: Samyang Corporation, Seoul 110-725 (KR)
(72) Inventor: YOON, Hye-Sung, 305-340 Daejeon (KR); KIM, Tae-Hun, 302-723 Daejeon (KR); KOH, Myoung-Seok, 301-781 Daejeon (KR); HONG, Chong-Taek, Yuseong-Gu, 305-729 Daejeon (KR); PAI, Chaul-Min, Yuseong-Gu, 305-755 Daejeon (KR); IM, Jung-Nam, Daejeon 305-333 (KR); SEO, Jang-Il, 305-503 Daejeon (KR)
(74) Representative: Wichmann, Hendrik
(86) International application number: PCT/KR2003/000150
(87) International publication number: WO 2004/003056

(56) References cited:
- EP-A2- 0 558 965
- US-A- 4 788 979
- US-A- 5 371 176
- US-A- 5 411 554
- US-B1- 6 423 818

## Description

### FIELD OF THE INVENTION

The present invention relates to block copolymers used for manufacturing surgical articles, and more particularly, to block copolymers prepared by copolymerization of ε-caprolactone and trimethylene carbonate to produce segmented copolymers which are then reacted with *p*-dioxanone. The block copolymers of the present invention are suitable for making articles for general medical and surgical operations, i.e., sutures that require mechanical and biological properties such as biocompatibility and absorption.

### BACKGROUND OF THE INVENTION

Absorbable biocompatible polymers are widely used to manufacture surgical articles. Surgical articles, especially sutures, should have adequate tensile strength and biocompatibility. In addition, depending on use; sutures should be absorbed by the living body with appropriate absorption rates. Sutures should be also easy to manage during surgical operations.

Good handling properties of sutures are often closely related to their flexibility. In other words, a suture having low flexibility cannot provide desired knot security, and thus is not suitable for applying to delicate body parts, such as suturing blood vessels. Sutures used in the operations on delicate body parts, such as blood vessels, require good flexibility.

Sutures with appropriate knot-security are required for effective wound closure. Previously, in order to improve knot-security, multiple knotting of poor knot secured sutures was required, which causes harmful side effects such as tissue reaction and infection. However, knots made by sutures having high flexibility can remain secure and provide excellent knot-security without multiple knotting.

In general, both good flexibility and knot-security are important physical properties of a suture. It is necessary that the suture should have a certain period of time to be absorbed. The period of time, so called absorption time, of the suture material indicates that the degradation of the suture material. In general, the surgical suture could be classified as 50% strength retention time according to its raw material in 7days, 10days, 14days, 21days, 28days. And the surgical suture can be used selectively depending on an application site of the body according to its absorbability.

In the beginning, the absorbable gut and collagen sutures were traditionally made from natural materials, and they often produced unfavorable tissue reactions. Further, it was difficult to predict a strength retention time, because the absorption rate of the natural materials was hard to predict. Therefore, attempts to apply synthetic polymers having biocompatibility to sutures have been made.

The previous synthetic absorbable sutures mostly contained polyglycolides greater than 60%. US Patent No. 5,431,679 describes block copolymers comprising polyglycolide, trimethylene carbonate, and caprolactone. Sutures made from these block copolymers show poor flexibility.

US Patent No. 5,854,383 discloses segmented copolymers of aliphatic polyesters comprising lactone monomers, glycolide, trimethylene carbonate, and ε-caprolactone. Although these segmented copolymers provide an improved flexibility compared to those of US Patent No. 5,431,679, they have a slower absorption rate and show poor knot-security.

US Patent No. 4,052,988 discloses the preparation of a *p*-dioxanone homopolymer and its use as an absorbable surgical suture. This synthetic suture exhibits outstanding mechanical and biological properties to make it a viable candidate to replace the natural sutures, such as surgical gut and collagen, for numerous applications. Furthermore, US Patent No. 5,047,048 discloses the preparation of copolymers of *p*-dioxanone and ε-caprolactone that provide improved flexibility compared to the *p*-dioxanone homopolymer. However, the suture comprised in *p*-dioxanone and ε-caprolactone showed too long 50% strength retention time as over 3 weeks and could not satisfy the desired flexibility.

US 6,423,818 discloses a photo curable, liquid polymers incorporating coumarin ester and groups into their molecular structure, which polymers are crosslinked upon a radiation with ultraviolet light by photo chemically allowed [2 + 2] cycle reaction among the chain ends, and which crosslinked polymers are useful for the preparation of medical devices, tissue engineerings caffolds, drag delivery systems and, in particular, in vivo preparations of implants in an open surgical procedure or laproscopiclaally.

US 4,788,979 discloses a bioabsorbable coating for a surgical article comprising a copolymer manufactured from the monomer caprolactone and at least one other copoylmerisable monomer. The surgical article can be a bioabsorbable suture or ligature.

US 5,411,554 discloses a cosmetic or prosetic surgical implant comprising an envelop filled with a liquid bioabsorbable polymer and a process for implanting a cosmetic or prosetic surgical implant involving the filling of an envelop with a liquid bioabsorbable polymer.

As described above, much effort has been put into improving the properties of sutures. However, the above mentioned patents and other researches have not produced suture materials having the properties of flexibility, knot security, and controlling of absorption properties, which are important requirements of sutures. Therefore, the present invention provides block copolymers with excellent knot security, flexibility, and appropriate absorption, which overcome the disadvantages of currently commercialized surgical materials.

### SUMMARY OF THE INVENTION

The present invention relates to novel block copolymers prepared by reacting segmented copolymers of ε-caprolactone and trimethylene carbonate with *p*-dioxanone monomers. The block copolymers of the present invention are well suited for surgical articles requiring high flexibility, excellent knot-security, and rapid absorption. The present invention also relates to methods for preparing such block copolymers.

### BRIEF DESCRIPTION OF THE DRAWING

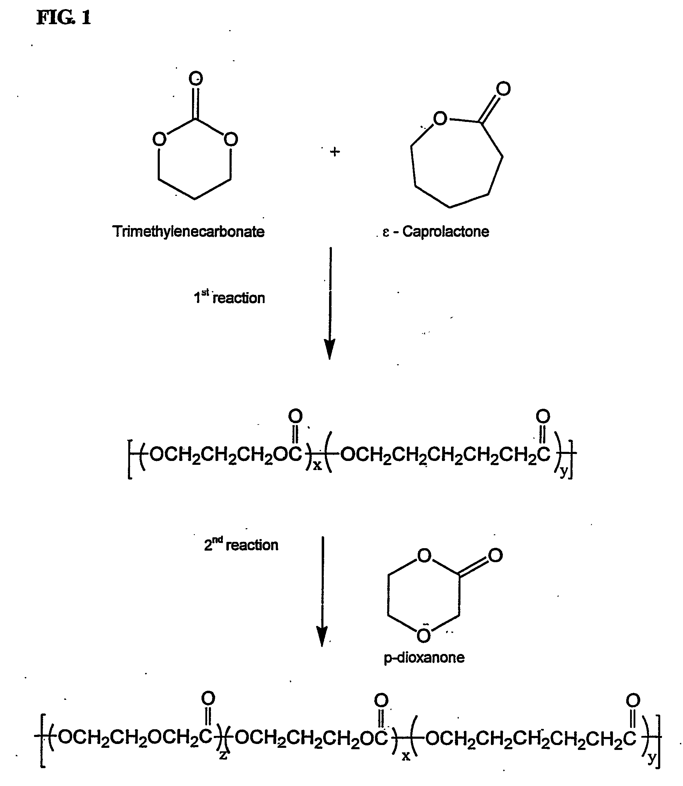

Fig. 1 illustrates a synthesis scheme for preparing the block copolymers of the present invention.

### DETAILED DESCRIPTION OF THE INVENTION

The present invention relates to block copolymers comprising *p*-dioxanone blocks and segmented copolymer blocks of ε-caprolactone and trimethylene carbonate.

The present invention also relates to a method for producing a block copolymer which comprises the steps of:
(a) polymerizing ε-caprolactone and trimethylene carbonate to produce a segmented copolymer block; and
(b) reacting the segmented copolymer block with *p*-dioxanone monomers to produce a block copolymer comprising *p*-dioxanone blocks and segmented copolymer blocks of ε-caprolactone and trimethylene carbonate.

The present invention is described in more detail hereunder.

One embodiment of the present invention is a block copolymer comprising a polydioxanone block and a segmented copolymer block comprising ε-caprolactone copolymerized with trimethylene carbonate. In the present invention, two types of blocks having different structures are advantageously combined to form a block copolymer.

The block copolymers of the present invention can be prepared as AB type diblock or ABA type triblock copolymers. AB type diblock copolymers can be prepared by polymerizing p-dioxanone monomer described as A with a segmented copolymer of ε-caprolactone and trimethylene carbonate described as B. ABA type triblock copolymers also can be prepared by using polydioxanone as the end blocks and the segmented copolymer of a ε-caprolactone and trimethylene carbonate as the center blocks.

The block copolymers of the present invention comprise polydioxanone as expressed by the following formula (1) and a segmented copolymer unit of ε-caprolactone and trimethylene carbonate as expressed by the following formula (2), 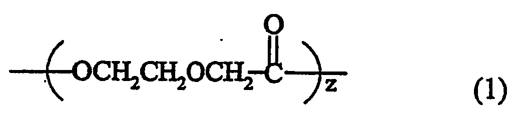 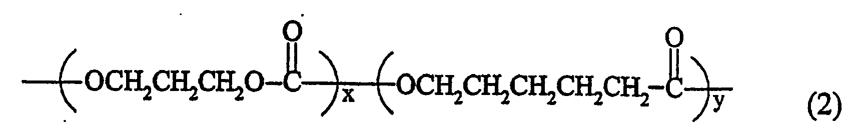 wherein x, y, and z represent an integer between 10 to 10⁴.

Fig. 1 represents a synthesis scheme of preparing the block copolymers of the present invention.

For example, the process for preparing the block copolymers of the present invention comprises two steps. First, copolymerization of ε-caprolactone and trimethylene carbonate is carried at a temperature of 120 to 180°C, preferably 150 to 170°C, to form a segmented copolymer. Second, the resulting segmented copolymer is then reacted with *p-*dioxanone monomers to produce the block copolymers of the present invention, as shown in Fig. 1. In other words, the segmented copolymer of formula (2) comprising ε-caprolactone and trimethylene carbonate obtained from the first copolymerization step is reacted with *p*-dioxanone monomers to produce a block copolymer in which polydioxanone of formula (1) and a segmented copolymer unit of formula (2) form blocks.

In a preferred embodiment of the present invention, the first copolymerization to produce the segmented copolymer of formula (2) is carried out at a temperature of 120 to 180°C, preferably 150 to 170°C, for 0.5 to 2 hours. In the second copolymerization, *p-*dioxanone is then added to the segmented copolymer at a temperature of 130 to 160°C and reacted for 1 to 3 hours. The reaction temperature is preferably lowered to 90°C and the reaction is preferably performed for 5 to 20 hours. Furthermore, the reaction temperature may be lowered to 80°C and the reaction performed for 3 to 5 days, preferably for 3 to 4 days.

In the second copolymerization step to produce the block copolymer, the reaction is preferably performed via multiple steps because *p*-dioxanone is likely to decompose at high temperature. Therefore, after the segmented copolymer is formed, the second reaction is preferably performed at a lower temperature.

The block copolymers of the present invention are synthesized in the presence of an organometal catalyst and an initiator, which are typically used in a ring opening polymerization. The organometal catalyst is preferably tin 2-ethylhexanoate, and is preferably present in the monomer mixture at a molar ratio of monomers (PTMC + PCL + PDO) to catalyst ranging from 10,000:1 to 100,000:1. The initiator used in the present invention is typically an alkanol such as diols, polyols or glycols, a hydroxyacid, or an amine, and is preferably present in the monomer mixture at a molar ratio of monomers to initiator ranging from about 500:1 to about 5,000:1. The polymerization reaction is carried out at appropriate temperature and for an appropriate period of time until the desired molecular weight and viscosity are achieved.

Depending on the initiator used in the synthesis, the block copolymers formed in the present invention can be diblock or triblock copolymers. Diblock copolymers of the present invention can be prepared by polymerizing p-dioxanone monomers with segmented copolymers of ε-caprolactone and trimethylene carbonate. Triblock copolymers of the present invention have polydioxanone as the end blocks and the segmented copolymer of ε-caprolactone and trimethylene carbonate as the center block. The initiator used for producing triblock copolymers of the present invention is a bifunctional initiator, such as diols having hydroxyl groups at both sides. For example, when ε-caprolactone and trimethylene carbonate are polymerized in the presence of diethylene glycol (hereafter referred to as "DEG") as an initiator, both hydroxyl groups of the DEG react with each of ε-caprolactone and trimethylene carbonate to form a segmented copolymer which may further react with p-dioxanone. p-dioxanone is then added and reacted at the both ends of the segmented copolymer to produce a triblock copolymer.

Preferably, the molar ratio of ε-caprolactone/trimethylene carbonate in the segmented copolymer as shown in the above formula (2) is within the range of 5:95 to 95:5, and more preferably within the range of 90:10 to 10:90. A higher molar ratio of trimethylene carbonate in the segmented copolymer correlates to higher flexibility, while a higher molar ratio of ε-caprolactone in the segmented copolymer correlates to a longer period of strength retention. Therefore, the molar ratio of ε-caprolactone/trimethylene carbonate can be controlled to provide various degrees of flexibility, strength retention, or absorption rate. The segmented copolymers of the present invention preferably have inherent viscosity within the range of 0.5 dL/g to 2.1 dL/g, more preferably 0.7 dL/g to 1.8 dL/g as measured in a 0.1 g/dL solution of hexafluoroisopropanol (HFIP) at 25°C.

Preferably, the content of polydioxanone as shown in the above formula (1) in the diblock copolymers of the present invention is within the range of 5 to 95 mole%, more preferably 60 to 90 mole%. When the content of p-dioxanone is below 5 mole%, the copolymer cannot be converted into the form of surgical suture through melt extrusion due to crystallinity of lower than 10%, and also it fails to obtain the desired strength and absorption rate. Preferably, the degree of crystallinity of the copolymer of the present invention is 10% or more, and more preferably 20 to 27%. Also, in the triblock copolymers of the present invention, the content of polydioxanone is within the range of 10 to 90 mole%. The content below 10mole% in the triblock copolymers may cause the same kind of disadvantages.

The copolymer of the present invention can be a random or block copolymer but the block copolymer is much more preferred. The crystallinity of the random copolymer is too low to have knot tensile strength of 10,000 psi or greater, which is required as a surgical suture.

The block copolymers of the present invention have inherent viscosity ranging from 0.8 dL/g to 4.0 dL/g, preferably 1.0 dL/g to 3.5 dL/g as measured in a 0.1 g/dL solution of hexafluoroisopropanol (HFIP) at 25°C. The block copolymers of the present invention have Young's modulus of about 180,000 psi or less, preferably 140,000 psi or less.

Since the block copolymers of the present invention are prepared by polymerization of the segmented copolymer with *p*-dioxanone monomer, the total crystalline domains are reduced in comparison to *p*-dioxanone homopolymers, whereby they provide for a low degree of crystallinity. In other words, the increased non-crystalline domains of block copolymers of the present invention have a low Young's modulus in comparison to the Young's modulus of a *p*-dioxanone homopolymer. The low Young's modulus of the block copolymers correlates with higher flexibility and more stable knot-security. Furthermore, the block copolymers have higher strength due to physical crosslinks between the non-crystalline domains. In addition, reduction in the degree of crystallinity is also correlated with faster absorption. Therefore, the block copolymers of the present invention have a low Young's modulus, high flexibility, and faster absorption rate while maintaining good strength.

The block copolymers of the present invention are useful for the preparation of implantable medical and surgical articles, especially absorbable surgical sutures, surgical screws, surgical staples, surgical clips, pins, anastomosis rings, surgical meshes, prosthetic devices, and the like. The block copolymers of the present invention can be injection-molded into surgical screws, surgical staples, surgical clips, pins, and anastomosis rings. The block copolymers of the present invention can be melt-extruded and drawn to produce filaments such as sutures. A surgical mesh can be prepared by knitting and/or weaving or non-weaving the filaments.

The block copolymers of the present invention are suitable for making surgical articles such as prosthetic devices, particularly surgical sutures that require a low Young's modulus and high knot-security. Additionally, the sutures made from the block copolymers of the present invention can be attached to needles. Among potential suture applications of the block copolymers of the present invention, monofilament sutures are preferred because they have less tissue drag and lower infection rate.

Monofilament sutures prepared from the block copolymers of the present invention have straight tensile strength of at least 45,000 psi, preferably 45,000 to 70,000 psi, and more preferably 49,000 to 53,000 psi. The knot-pull strength of monofilament sutures prepared from the block copolymers of the present invention is at least 25,000 psi, preferably 30,000 to 50,000 psi, and more preferably 30,000 to 33,000 psi. The Young's modulus of monofilament sutures prepared from the block copolymers of the present invention is 180,000 psi or less, preferably 30,000 to 180,000 psi. Sutures prepared from the block copolymers of the present invention have a 50% strength retention time of 7 to 10 days, thus suitable in the situations requiring rapid absorption.

As described above, the block copolymers of the present invention prepared by reacting p-dioxanone monomers with segmented copolymers of ε-caprolactone and trimethylene carbonate are suitable for sutures and have excellent properties such as a low Young's modulus, high flexibility, high knot strength, and high knot-security The block copolymers of the present invention are also useful for other materials requiring biocompatibility and rapid absorption.

The block copolymers obtained by the present invention have excellent strength, flexibility, and absorbability, and are suitable for medical articles such as surgical sutures, surgical screws, surgical staples, surgical clips, pins, anastomosis rings, surgical meshes, prosthetic devices, and the like. The block copolymers obtained by the present invention are especially useful for monofilament synthetic absorbable sutures. Furthermore, the block copolymers of the present invention have high strength, flexibility, excellent knot-security, and a 50% strength retention time of from 7 to 10 days, and so are useful for surgical articles which demand rapid absorption.

The following Examples and Comparative Examples are intended to further illustrate the present invention without limiting its scope.

The monomers and block copolymers used in the Examples and Comparative Examples were analyzed by the following methods: NMR (Bruker AVANCE DPX 400) and GC (HP5890) for chemical structure and purity thereof; a viscometer (Cannon) for inherent viscosity as measured in a 0.1 g/dL solution of hexafluoroisopropanol (HFIP) at 25°C; X-ray diffraction (Rigaku Tenki RADB) for the degree of crystallinity. In vitro strength change was measured after storing the block copolymers in phosphate buffer solution (pH=7.27) at 37°C over time.

The block copolymer was molten, spun, drawn, and annealed by using conventional extrusion techniques to prepare sutures.

### Methods for measuring the physical properties of sutures - knot security

Knot security was measured in terms of the knot slippage ratio. A surgeon's knot (2=1=1) was selected for the knot tying method. The knotted sutures were placed on a tensile strength tester and pulled apart until knot breakage occurred or the knot slipped. After the measurements by ten times, the ratio of the number of knots slipped to the total number of knots tied indicates the knot slippage ratio. Thus, the less the ratio is, the better the knot security of the suture is.

### Methods for measuring the physical properties of sutures - flexibility

The flexibility data of sutures are based on the Young's moduli derived from measuring linear tensile strength.

**Table 1**

| Physical properties | Method |
|---|---|
| Straight tensile strength | ASTM D-2256 |
| | Used machine: Instron |
| | Length of sample: 130 mm |
| | Measuring Rate: 130 mm/min |
| Knot-pull strength | EP (European Pharmacopeias) |
| | (Monofilament suture) |
| | Used machine: Instron |
| | Length of sample: 100 mm |
| | Measuring Rate: 100 mm/min |
| Diameter, mm | EP regulation, Diameter |
| Young's modulus | Used machine: Instron |
| | Stress-strain curve |
| Knot-security | Used machine: Instron |
| | Type ofknots: Surgeon's knot (2=1=1) |
| | Length of sample: 5 mm |
| | Measuring Rate: 500mm/min |
| | Standard for determination: knot slippage ratio = (sliding knotted samples no./10 knotted samples) x 100 |

### Example 1

### Preparation of diblock copolymer comprising ε-caprolactone/trimethylene carbonate at 10/90 by mole and 90 mole% of p-dioxanone

To a dried flask were added 138.6 g of trimethylene carbonate and 15.4 g of ε-caprolactone. The contents of the reaction flask, fitted with a mechanical stirrer, were held and dried under vacuum. After drying, to the reaction mixture were added 0.8 mL of tin 2-ethylhexanoate (0.63 M solution in toluene) and 1.8 g of lauryl alcohol by syringe. The reaction mixture was reacted in an oil bath while maintaining the temperature of 160°C for 2 hours under a nitrogen atmosphere. The reactor was purged with nitrogen and 1346.4 g of *p*-dioxanone was added while stirring. The temperature of reaction mixture was lowered to 150°C and kept for 1 hour, and then lowered to 90°C and kept for 16 hours, and further lowered to 80°C and kept for 72 hours. A block copolymer was isolated, pulverized, and dried under vacuum to remove any unreacted monomer.

The composition obtained is polydioxanone (PDO)/polytrimethylene carbonate (PTMC)/polycaprolactone (PCL), (89.9/9.0/1.1), as determined by ¹H-NMR. The degree of crystallinity of the copolymer was 23 %, as determined by X-ray diffraction. The inherent viscosity of the diblock copolymer was 2.3dL/g as measured in a 0.1 g/dL solution of hexafluoroisopropanol (HFIP) at 25°C. The properties of the block copolymer are summarized in Table 2 below.

### Example 2

### Preparation of diblock copolymer comprising ε-caprolactone/trimethylene carbonate at 25/75 by mole and 90 mole% of p-dioxanone

To a dried flask were added 112.2 g of trimethylene carbonate and 41.8 g of ε-caprolactone. The contents of the reaction flask, fitted with a mechanical stirrer, were held and dried under vacuum. After drying, to the reaction mixture were added 0.8 mL of tin 2-ethylhexanoate (0.63 M solution in toluene) and 1.8 g of lauryl alcohol by syringe. The reaction mixture was reacted in an oil bath while maintaining the temperature of 160°C for 2 hours under a nitrogen atmosphere. The reactor was purged with nitrogen and 1346.4 g of *p*-dioxanone was added thereto while stirring. The temperature of the reaction mixture was lowered to 150°C and kept for 1 hour, and then lowered to 90°C and kept for 16 hours, and further lowered to 80°C and kept for 72 hours. A block copolymer was isolated, pulverized, and dried under vacuum to remove any unreacted monomer.

The composition obtained is polydioxanone (PDO)/polytrimethylene carbonate (PTMC)/polycaprolactone (PCL), (89/8.5/2.5), as determined by ¹H-NMR. The degree of crystallinity of the copolymer was 25 %, as determined by X-ray diffraction. The inherent viscosity of the diblock copolymer was 2.9 dL/g as measured in a 0.1 g/dL solution of hexafluoroisopropanol (HFIP) at 25°C. The properties of the block copolymer are summarized in Table 2.

### Example 3

### Preparation of diblock copolymer comprising ε-caprolactone/trimethylene carbonate at 75/25 by mole and 90 mole% of p-dioxanone

To dried flask were added 37.4 g of trimethylene carbonate and 125.5 g of ε-caprolactone. The contents of the reaction flask, fitted with a mechanical stirrer, were dried under vacuum. After drying, to the reaction mixture were added 1 mL of tin 2-ethylhexanoate (0.63 M solution in toluene) and 2 g of lauryl alcohol via syringe. The reaction mixture was reacted in an oil bath while maintaining the temperature of 160°C for 2 hours under a nitrogen atmosphere. The reactor was purged with nitrogen and 1346.4 g of *p*-dioxanone was added thereto while stirring. The temperature of the reaction mixture was lowered to 150°C and kept for 1 hour, and then lowered to 90°C and kept for 16 hours, and further lowered to 80°C and kept for 72 hours. A block copolymer was isolated, pulverized, and dried under vacuum to remove any unreacted monomer.

The composition of the polydioxanone (PDO)/polytrimethylene carbonate (PTMC)/polycaprolactone (PCL) obtained was 89/3.5/7.5 as determined by ¹H-NMR. The degree of crystallinity of the copolymer was 27%, which was determined by X-ray diffraction. The inherent viscosity of the diblock copolymer was 2.3 dL/g as measured in a 0.1 g/dL solution of hexafluoroisopropanol (HFIP) at 25°C. The properties of the formed block copolymer are summarized in Table 2.

### Example 4

### Preparation of diblock copolymer comprising ε-caprolactone/trimethylene carbonate at 50/50 by mole and 80 mole% of p-dioxanone

To a dried flask were added 204 g of trimethylene carbonate and 228 g of ε-caprolactone. The contents of the reaction flask, fitted with a mechanical stirrer, were dried under vacuum. After drying, to the reaction mixture were added 2.29 mL of tin 2-ethylhexanoate (0.63 M solution in toluene) and 4 g of lauryl alcohol via syringe. The reaction mixture was reacted in an oil bath while maintaining the temperature of 160°C for 2 hours under a nitrogen atmosphere. The reactor was purged with nitrogen and 1632 g of *p*-dioxanone was added thereto while stirring. Further reactions were performed in the same manner as Example 1. The properties of the formed block copolymer are summarized in Table 2 below.

### Example 5

### Preparation of diblock copolymer comprising ε-caprolactone/trimethylene carbonate at 25/75 by mole and 80 mole% of p-dioxanone

To a dried flask were added 306 g of trimethylene carbonate and 114.1 g of ε-caprolactone. The contents of the reaction flask, fitted with a mechanical stirrer, were dried under vacuum. After drying, to the reaction mixture were added 2.29 mL of tin 2-ethylhexanoate (0.63 M solution in toluene) and 4 g of lauryl alcohol via syringe. The reaction mixture was reacted in an oil bath while maintaining the temperature of 160°C for 2 hours under a nitrogen atmosphere. The reactor was purged with nitrogen and 1632 g of *p*-dioxanone was added thereto while stirring. Further reactions were performed in the same manner as Example 1. The properties of the formed block copolymer are summarized in Table 2 below.

### Example 6

### Preparation of diblock copolymer comprising ε-caprolactone/trimethylene carbonate at 75/25 by mole and 80 mole% of p-dioxanone

To a dried flask were added 102 g of trimethylene carbonate and 342.3 g of ε-caprolactone. The contents of the reaction flask, fitted with a mechanical stirrer, were dried under vacuum. After drying, to the reaction mixture were added 2.29 mL of tin 2-ethylhexanoate (0.63 M solution in toluene) and 4 g of lauryl alcohol via syringe. The reaction mixture was reacted in an oil bath while maintaining the temperature of 160°C for 2 hours under a nitrogen atmosphere. The reactor was purged with nitrogen and 1632 g of *p*-dioxanone was added thereto while stirring. Further reactions were performed in the same manner as Example 1. The properties of the formed block copolymer are summarized in Table 2 below.

### Example 7

### Preparation of triblock copolymer comprising p-dioxanone as the end blocks and a segmented copolymer of trimethylene carbonate and ε-caprolactone as the center block, wherein the composite ratio is 45/(7/3)/45 by mole

To a dried flask were added 112.2 g of trimethylene carbonate and 41.8 g of ε-caprolactone. The contents of the reaction flask, fitted with a mechanical stirrer, were dried under vacuum. After drying, to the reaction mixture were added 1.8 mL of tin 2-ethylhexanoate (0.63 M solution in toluene) and 1.8 g of diethylene glycol (DEG) via syringe. The reaction mixture was reacted in an oil bath while maintaining the temperature of 170°C for 1 hour. The reactor was purged with nitrogen and the temperature of the reaction mixture was lowered to 160°C, and 1346.4 g of *p*-dioxanone was added thereto while stirring. The reaction temperature was maintained at 160°C for 1 hour under a nitrogen atmosphere. The temperature of the reaction mixture was lowered to 90°C and kept for 16 hours, and then lowered to 80°C and kept for 72 hours. The block copolymer was isolated, pulverized, and dried under vacuum to remove any unreacted monomer.

The composition of the polydioxanone (PDO)/polytrimethylene carbonate (PTMC)/polycaprolactone (PCL)/polydioxanone (PDO) obtained was 44/8.0/3.0/45 as determined by ¹H-NMR. The inherent viscosity of the triblock copolymer was measured in a 0.1 g/dL solution of hexafluoroisopropanol (HFIP) at 25°C. The properties of the block copolymer are summarized in Table 2.

**Table 2**

| Polymer | Composition (PDO/PTMC/PCL) | Inherent viscosity (dL/g) | Crystallinity (%) |
|---|---|---|---|
| Example 1 | 89.9/9.0/1.1 | 2.3 | 23 |
| Example 2 | 89/8.5/2.5 | 2.9 | 25 |
| Example 3 | 89/3.5/7.5 | 2.3 | 27 |
| Example 4 | 79.2/10.4/10.4 | 1.8 | 20 |
| Example 5 | 79/15.6/5.4 | 2.3 | 23 |
| Example 6 | 79.1/5.0/15.9 | 2.0 | 25 |
| Example 7* | 44/(8/3)/45 | 2.0 | 26 |
| Control** | 100 | 2.5 | 32 |
| * Example 7 : Triblock copolymer comprising PDO/(PTMC/PCL)/PDO | | | |
| **Control : PDO homopolymer | | | |

As shown in Table 2, both diblock copolymers and triblock copolymers of the present invention have comparative inherent viscosities in the range of 1.8 to 2.9 dL/g, and crystallinities of 20 to 27% that are about 5 to 12% lower than those of PDO homopolymers. When the amount of PDO is the same, the increase in the amount of PTMC correlates with reduced crystallinity and the increase in the amount of PCL correlates with increased crystallinity.

### Comparative Example 1

### Preparation of random copolymer comprising ε -caprolactone, trimethylene carbonate and p-diozanone

To a dried flask were added 306 g of trimethylene carbonate, 114.1 g of ε-caprolactone, and 612 g of *p*-dioxanone. The contents of the reaction flask, fitted with a mechanical stirrer, were dried under vacuum. After drying, to the reaction mixture were added 0.8 mL of tin 2-ethylhexanoate (0.63 M solution in toluene) and 1.8 g of lauryl alcohol via syringe. The reaction mixture was reacted in an oil bath while maintaining the temperature of 160°C for 2 hours. The reactor was purged with nitrogen and the temperature of the reaction mixture was lowered to 150°C and kept for 1 hour under a nitrogen atmosphere, and then lowered to 90°C and kept for 16 hours. The copolymer was isolated, pulverized, and dried under vacuum to remove any unreacted monomer.

The composition of the polydioxanone (PDO)/polytrimethylene carbonate (PTMC)/polycaprolactone (PCL) obtained was 62/38/10 as determined by ¹H-NMR. The degree of crystallinity of the copolymer was 15% which was determined by X-ray diffraction. The inherent viscosity of the random copolymer was 1.7 dL/g. The crystallinity of the random copolymer was too low to be used as a suture due to the low knot strength of 10,000 psi.

### Comparative Example 2

### Preparation of block copolymer comprising p-dioxanone/ε-caprolactone at 90/10 by mole

To a dried 500 mL flask was added 41.0 g of ε-caprolactone. The contents of the reaction flask, fitted with a mechanic stirrer, were dried under vacuum. After drying, to the reaction mixture were added 0.34 mL of tin 2-ethylhexanoate (0.63 M solution in toluene) and 2 g of lauryl alcohol via syringe. The reaction mixture was reacted in an oil bath while maintaining the temperature of 160°C for 1 hour. The reactor was purged with nitrogen and the temperature of the reaction mixture was lowered to 110°C, and then 265.2 g of *p-*dioxanone was added thereto while stirring. The reaction temperature was maintained at 110°C for 4 hours, and then lowered to 90°C and kept for 24 hours, and further lowered to 80°C and kept for 72 hours. The block copolymer was isolated, pulverized, and dried under vacuum to remove any unreacted monomer.

The composition of the polydioxanone (PDO)/ polycaprolactone (PCL) obtained was 88.8/10.2 as determined by ¹H-NMR. The degree of crystallinity of the copolymer was 26.3% which was determined by X-ray diffraction. The inherent viscosity of the random copolymer was 2.21 dL/g. Crystallinity and inherent viscosity of the copolymer of Comparative example 2 are similar to block copolymers of the present invention but, breaking strength retention of block copolymers of the present invention is much faster than that of the copolymer of Comparative Example 2.(confer Example 10).

### Example 8

### Preparation of monofilament sutures using block copolymers

The block copolymers from Examples 1, 2, 3, 5, 6 and 7 were extruded into monofilaments under the following conditions; see Table 3, to compare copolymer properties. The copolymer properties are summarized in Table 4.

**Table 3**

| Melt Extrusion | | |
|---|---|---|
| Category | Unit | Condition |
| Extruder screw | rpm | 6 |
| Pump | rpm | 11 |
| Barrel temperature, 1 zone | °C | 175 |
| Barrel temperature, 2 zone | °C | 177 |
| Barrel temperature, 3 zone | °C | 179 |
| Pump temperature | °C | 179 |
| Pump melt temperature | °C | 180 |
| Spinneret melt temperature | °C | 180 |
| Barrel Pressure | Kgf/cm² | 70 |
| Spinneret Pressure | Kgf/cm² | 60 |
| Pump size | cc/rev | 2.0 |
| Quench bath temperature | °C | 22 |

| Stretching & Orientation | | |
|---|---|---|
| Draw oven temperature (1^{st}/2^{nd}/3^{rd}) | °C | 90/95/95 |
| First godet | mpm | 5.4 |
| Second godet | mpm | 26.4 |
| Third godet | mpm | 27.9 |
| Winding speed | rpm | 25 |
| Draw ratio | | 4.63 |

**Table 4**

| Block copolymer | Example 1 | Example 2 | Example 3 | Example 5 | Example 6 | Example 7 |
|---|---|---|---|---|---|---|
| Composition (PDO/PTMC/ PCL) | 89.9/9.0/1.1 | 89/8.5/2.5 | 89/3.5/7.5 | 79/15.6/5. 4 | 79.1/5/15.9 | 44/8/3/45 |
| Diameter | 0.546mm | 0.548 mm | 0.534 mm | 0.550 mm | 0.530 mm | 0.549 mm |
| Knot pull strength | 35,000psi | 32,000 psi | 30,000 psi | 30,000 psi | 31,000 psi | 33,000 psi |
| Straight tensile strength | 58,000psi | 53,000 psi | 50,000 psi | 49,000 psi | 51,000 psi | 53,000 psi |
| Young's Modulus | 100,000psi | 120,000 psi | 150,000 psi | 90,000 psi | 110,000 psi | 130,000 psi |
| Elongation | 67% | 64% | 70% | 68% | 72% | 66% |

As shown in Table 4 above, the block copolymer of the present invention has the straight tensile strength of 49,000 psi or greater, high knot strength of at least 30,000, Young's Modulus of 150,000 psi or less, and excellent flexibility. Also when the amount of PDO was the same, a higher molar ratio of trimethylene carbonate in the segmented copolymer correlates to higher flexibility.

### Example 9

### Property comparison of the present invention's copolymers, Comparative Example 2's copolymer, and commercial monofilament suture

The copolymer of Example 1 (PDO/PTMC/PCL), the copolymer of Example 4 (PDO/PTMC/PCL), and the copolymer of Comparative Example 2 (PDO-PCL) were extruded into a monofilament under the conditions listed in Table 3 to compare copolymer properties such as Young's Modulus and knot-security. The commercial monofilament used was MONOCRYL™ (Ethicon Inc.), made from glycolide and ε-caprolactone.

**Table 5.**

| Monofilament | Example 1 | Example 4 | Comparative | MONOCRYL™ |
|---|---|---|---|---|
| Young's Modulus(psi) | 100,000 | 90,000 | 200,000 | 230,000 |
| Knot-security(%) | 30 | 50 | 100 | 80 |

As shown in Table 5, the block copolymers of the present invention exhibit not only significant reduction in Young's Modulus as compared to MONOCRYL™ and Comparative example 2(PDO-PCL copolymer) but also higher flexibility. Furthermore, the block copolymers of the present invention show better knot-security (3-5 out of 10 knots failed) than MONOCRYL™ (8 knots failed) and Comparative example 2(10 knots failed).

### Example 10

### Comparison in breaking strength retention of the present invention's block copolymers, Comparative Example 2's copolymer, and commercial monofilament

The change of strength retention in vitro was measured after storage in a phosphate buffer, pH 7.27 at 37°C by comparison with the initial strength. The results are summarized in Table 6.

**Table 6: Breaking strength retention depending on a storage period**

| Monofilament | Example 1 | Example 3 | Comparative Example 2 | MONOCRYL™ |
|---|---|---|---|---|
| Initial | 100 | 100 | 100 | 100 |
| After 7 days | 60 | 77 | 90 | 70 |
| After 10 days | 49 | 55 | 90 | 60 |
| After 14 days | 30 | 40 | 80 | 55 |

As shown in Table 6, the 50% strength retention period of the block copolymers of the present invention was 10 days, which was much faster than that of the copolymer of Comparative Example 2 or MONOCRYL™. Furthermore, the increase in the amount of PTMC, when the amount of PDO was the same, correlates to reduction in the 50% strength retention period as shown in Examples 1 and 3.

The above description will enable one skilled in the art to make the block copolymers of the present invention by reacting p-dioxanone monomers with the segmented copolymers of ε-caprolactone and trimethylene carbonate, and to thereby make sutures have excellent properties such as low Young's modulus, high flexibility, high knot strength, and high knot-security. Although they are described to show functionality of the block copolymers of the present invention, these descriptions are not intended to be exhaustive. It will be immediately apparent to one skilled in the art that various modifications may be made without departing from the scope of the invention, which is limited only by the following claims and their functional equivalents.

## Claims

1. An AB type diblock copolymer comprising a *p*-dioxanone A block, and a segmented copolymer B block of ε-caprolactorie and trimethylene carbonate, wherein the inherent viscosity of said AB type diblock copolymer is in the range of 0.8 to 4.0 dL/g as measured in a 0.1 g/dL solution of hexafluoroisopropanol at 25°C.

2. The diblock copolymer of claim 1, wherein the content of said *p*-dioxanone A block is within the range of 5 to 95 mole%.

3. The diblock copolymer of claim 2, wherein the content of said *p*-dioxanone block is within the range of 60 to 90 mole%.

4. The diblock copolymer of claim 1, wherein the content of said segmented copolymer B block of ε-caprolactone and trimethylene carbonate is within the range of 5 to 95 mole%.

5. The diblock copolymer of claim 1, wherein the molar ratio of ε-caprolactone/trimethylene carbonate in said segmented copolymer B block is within the range of 5/95 to 95/5.

6. The diblock copolymer of claim 5, wherein the molar ratio of ε-caprolactone/trimethylene carbonate in said segmented copolymer B block is within the range of 10/90 to to 90/10.

7. The diblock copolymer of claim 1, wherein the inherent viscosity of said block copolymer is in the range of 1.0 to 3.5 dL/g as measured in a 0.1 g/dL solution of hexafluoroisopropanol at 25°C.

8. The diblock copolymer of claim 1 which has Young's modulus of 180,000 psi or less.

9. The diblock copolymer of claim 1, wherein the degree of crystallinity of said block copolymer is 10% or more.

10. The diblock copolymer of claim 9, wherein the degree of crystallinity of said block copolymer is 20 to 27 %.

11. An AB type diblock copolymer according to claim 1, wherein the content of said *p*-dioxanone A block is within the range of 5 to 95 mole%, the molar ratio of ε-caprolactone/trimethylene carbonate in said segmented copolymer B block is within the range of 5/95 to 95/5.

12. A surgical article comprising an AB type diblock copolymer comprising a *p*-dioxanone A block, and a segmented copolymer B block of ε-caprolactone and trimethylene carbonate, wherein the inherent viscosity of said block copolymer is in the range of 0.8 to 4.0 dL/g as measured in a 0.1 g/dL solution of hexafluoroisopropanol at 25°C.

13. The surgical article of claim 12, wherein the surgical article is a member selected from the group consisting of surgical sutures, surgical screws, surgical staples, surgical clips, pins, anastomosis rings, surgical meshes, and prosthetic devices.

14. The surgical article of claim 13, wherein said surgical suture is attached to needles.

15. The surgical article of claim 13, wherein the straight tensile strength of said surgical suture is within the range of 45,000 to 70,000 psi.

16. The surgical article of claim 13, wherein the knot-pull strength of said surgical suture is within the range of 30,000 to 50,000 psi.

17. The surgical article of claim 13, wherein the Young's modulus of said surgical suture is in the range of 30,000 to 180,000 psi.

18. A surgical article according to claim 12, wherein the content of said *p*-dioxanone A block is within the range of 5 to 95 mole%, and the molar ratio of ε-caprolactone/trimethylene carbonate in said segmented copolymer B block is within the range of 5/95 to 95/5.

19. The surgical article of claim 18, wherein the surgical article is a member selected from the group consisting of surgical sutures, surgical screws, surgical staples, surgical clips, pins, anastomosis rings, surgical meshes, and prosthetic devices.

20. The surgical article of claim 18, has straight tensile strength within the range of 45,000 to 70,000 psi, knot-pull strength within the range of 30,000 to 50,000 psi, and Young's modulus within the range of 30,000 to 180,000 psi.

21. An ABA type triblock copolymer comprising a *p*-dioxanone A block, and a segmented copplymer B block comprising ε-caprolactone and trimethylene carbonate, wherein the inherent viscosity of said block copolymer is in the range of 0.8 to 4.0 dL/g, as measured in a 0.1 g/dL solution of hexafluoroisopropanol at 25°C.

22. The triblock copolymer of claim 21, wherein the content of said *p*-dioxanone A block is within the range of 10 to 90 mole%.

23. The triblock copolymer of claim 21, wherein the content of said segmented copolymer B block of ε-caprolactone and trimethylene carbonate is within the range of 10 to 90 mole%.

24. The triblock copolymer of claim 21, wherein the molar ratio of ε-caprolactone/trimethylene carbonate in said segmented copolymer B block is within the range of 5/95 to 95/5.

25. The triblock copolymer of claim 24, wherein the molar ratio of ε-caprolactone/trimethylene carbonate in said segmented copolymer B block is within the range of 10/90 to 90/10.

26. The triblock copolymer of claim 21, wherein the inherent viscosity of said block copolymer is in the range of from 1.0 to 3.5 dL/g, as measured in a 0.1 g/dL solution of hexafluoroisopropanol at 25°C.

27. The triblock copolymer of claim 21 which has Young's modulus of 180,000 psi or less.

28. The triblock copolymer of claim 21, wherein the degree of crystallinity of said block copolymer is 10% or more.

29. The triblock copolymer of claim 28, wherein the degree of crystallinity of said block copolymer is 20 to 27 %.

30. An ABA type triblock copolymer according to claim 21, wherein the content of said *p*-dioxanone A block is within the range of 10 to 90 mole%, the molar ratio of ε-caprolactone/trimethylene carbonate in said segmented copolymer B block is within the range of 5/95 to 95/5.

31. A surgical article comprising an ABA type triblock copolymer comprising a *p-*dioxanone A block, and a segmented copolymer B block comprising ε-caprolactone and trimethylene carbonate, wherein the inherent viscosity of said block copolymer is in the range of 0.8 to 4.0 dL/g as measured in a 0.1 g/dL solution of hexafluoroisopropanol at 25°C.

32. The surgical article of claim 31, wherein the surgical article is a member selected from the group consisting of surgical sutures, surgical screws, surgical staples, surgical clips, pins, anastomosis rings, surgical meshes, and prosthetic devices.

33. The surgical article of claim 31, wherein said surgical suture is attached to needles.

34. The surgical article of claim 31, wherein the straight tensile strength of said surgical suture is within the range of 45,000 to 70,000 psi.

35. The surgical article of claim 31, wherein the knot-pull strength of said surgical suture is within the range of 30,000 to 50,000 psi.

36. The surgical article of claim 31, wherein the Young's modulus of said surgical suture is in the range of 30,000 to 180,000 psi.

37. A surgical article according to claim 31, wherein the content of said *p*-dioxanone A block is within the range of 10 to 90 mole%, and the molar ratio of ε-caprolactone/trimethylene carbonate in said segmented copolymer B block is within the range of 5/95 to 95/5.

38. The surgical article of claim 37, wherein the surgical article is a member selected from the group consisting of surgical sutures, surgical screws, surgical staples, surgical clips, pins, anastomosis rings, surgical meshes, and prosthetic devices.

39. The surgical article of claim 37 has straight tensile strength within the range of 45,000 to 70,000 psi, knot-pull strength within the range of 30,000 to 50,000 psi, and Young's modulus within the range of 30,000 to 180,000 psi.

40. A process for preparing a block copolymer comprising the steps of:
(a) polymerizing ε-caprolactone and trimethylene carbonate to produce a segmented copolymer block wherein the molar ratio of ε-caprolactone/trimethylene carbonate in said segmented copolymer is within the range of 10/90 to 90/10; and
(b) reacting the segmented copolymer block with *p*-dioxanone monomers wherein the content of said *p*-dioxanone is within the range of 5 to 95 mole% to produce a block copolymer comprising *p*-dioxanone blocks and segmented copolymer blocks of ε-caprolactone and trimethylene carbonate.

41. An AB or ABA type block copolymer comprising *p*-dioxanone A blocks, and segmented copolymer B blocks of ε-caprolactone and trimethylene carbonate prepared from the process of claim 40.

42. A block copolymer according to claim 1 or 2 comprising an A block of polydioxanone represented by formula (1) and a B block comprising a segmented copolymer of ε-caprolactone and trimethylene carbonate, said B block is represented by formula (2), 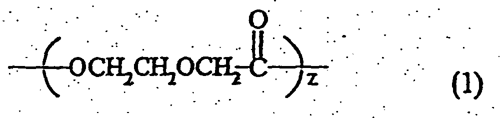 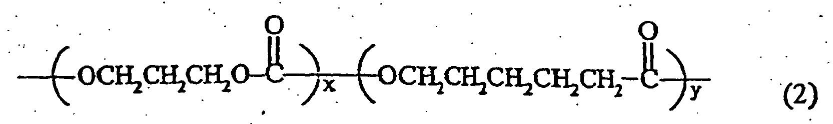 wherein x, y, and z represents an integer between 10 to 10⁴, and the inherent viscosity of said block copolymer is in the range of 0:8 to 4.0 dL/g, as measured in a 0.1 g/dL solution of hexafluoroisopropanol at 25°C.

43. The block copolymer of claim 42 which is a block copolymer containing 5 to 95 mole% of said A blocks and 5 to 95 mole% of said B blocks.

44. The block copolymer of claim 43 which is a block copolymer containing 10 to 90 mole% of said A blocks and 10 to 90 mole% of said B blocks.

45. The block copolymer of claim 42, wherein the degree of crystallinity of said block copolymer is 10% or more.

46. The block copolymer of claim 45, wherein the degree of crystallinity of said block copolymer is 10 to 27 %.

## Patentansprüche

1. Ein Diblock-Copolymer des Typs AB umfassend einen *p*-Dioxanon-A-Block und einen Segment-Copolymer-B-Block aus ε-Caprolacton und Trimethylencarbonat, wobei die inhärente Viscosität des Diblock-Copolymers des Typs AB im Bereich von 0,8 bis 4,0 dL/g ist, wie gemessen in einer 0,1 g/dL-Lösung aus Hexafluorisopropanol bei 25 °C.

2. Das Diblock-Copolymer nach Anspruch 1, wobei der Gehalt an dem *p*-Dioxanon-A-Block innerhalb des Bereichs von 5 bis 95 mol% ist.

3. Das Diblock-Copolymer nach Anspruch 2, wobei der Gehalt an dem *p*-Dioxanon-Block innerhalb des Bereichs von 60 bis 90 mol% ist.

4. Das Diblock-Copolymer nach Anspruch 1, wobei der Gehalt an dem Segment Copolymer-B-Block aus ε-Caprolacton und Trimethylencarbonat innerhalb des Bereichs von 5 bis 95 mol% ist.

5. Das Diblock-Copolymer nach Anspruch 1, wobei das Molverhältnis von ε-Caprolacton/Trimethylencarbonat in dem Segment-Copolymer-B-Block im Bereich von 5/95 bis 95/5 ist.

6. Das Diblock-Copolymer nach Anspruch 5, wobei das Molverhältnis von ε-Caprolacton/Trimethylencarbonat in dem Segment-Copolymer-B-Block im Bereich von 10/90 bis 90/10 ist.

7. Das Diblock-Copolymer nach Anspruch 1, wobei die inhärente Viskosität des Block-Copolymers im Bereich von 1,0 bis 3,5 dL/g ist, wie gemessen in einer 0,1 g/dL-Lösung aus Hexafluorisopropanol bei 25 °C.

8. Das Diblock-Copolymer nach Anspruch 1, welches einen Youngschen Elastizitätsmodul von 180.000 psi oder weniger hat.

9. Das Diblock-Copolymer nach Anspruch 1, wobei der Grad an Kristallinität des Block-Copolymers 10 % oder mehr ist.

10. Das Diblock-Copolymer nach Anspruch 9, wobei der Grad an Kristallinität des Block-Copolymers 20 bis 27 % ist.

11. Ein Diblock-Copolymer des Typs AB gemäß Anspruch 1, wobei der Gehalt an dem *p-*Dioxanon-A-Block im Bereich von 5 bis 95 mol% ist, das Molverhältnis von ε-Caprolacton/Trimethylencarbonat in dem Segment-Copolymer-B-Block im Bereich von 5/95 bis 95/5 ist.

12. Ein chirurgischer Gegenstand umfassend ein Diblock-Copolymer des Typs AB umfassend einen *p*-Dioxanon-A-Block und einen Segment-Copolymer-B-Block aus ε-Caprolacton und Trimethylencarbonat, wobei die inhärente Viskosität des Block-Copolymers im Bereich von 0,8 bis 4,0 dL/g ist, wie gemessen in einer 0,1 g/dL-Lösung aus Hexafluorisopropanol bei 25 °C.

13. Der chirurgische Gegenstand nach Anspruch 12, wobei der chirurgische Gegenstand ausgewählt ist aus der Gruppe bestehend aus chirurgischen Fäden, chirurgischen Schrauben, chirurgischen Klemmen, chirurgischen Klammern, Stiften, Anastomosenringen, Netzen und prothetischen Vorrichtungen.

14. Der chirurgische Gegenstand nach Anspruch 13, wobei der chirurgische Faden an Nadeln befestigt ist.

15. Der chirurgische Gegenstand nach Anspruch 13, wobei die geradlinige Reißfestigkeit des chirurgischen Fadens im Bereich von 45.000 bis 70.000 psi ist.

16. Der chirurgische Gegenstand nach Anspruch 13, wobei die Knotenzugstärke des chirurgischen Fadens im Bereich von 30.000 bis 50.000 psi ist.

17. Der chirurgische Gegenstand nach Anspruch 13, wobei der Youngsche Elastizitätsmodul des chirurgischen Fadens im Bereich von 30.000 bis 180.000 psi ist.

18. Ein chirurgischer Gegenstand nach Anspruch 12, wobei der Gehalt an dem *p-*Dioxanon-A-Block im Bereich 5 bis 95 mol% ist und das Molverhältnis von ε-Caprolacton/Trimethylencarbonat in dem Segment-Copolymer-B-Block im Bereich von 5/95 bis 95/5 ist.

19. Der chirurgische Gegenstand nach Anspruch 18, wobei der chirurgische Gegenstand ausgewählt ist aus der Gruppe bestehend aus chirurgischen Fäden, chirurgischen Schrauben, chirurgischen Klammern, chirurgischen Klemmen, Stiften, Anastomosenringen, chirurgischen Netzen und prothetischen Vorrichtungen.

20. Der chirurgische Gegenstand nach Anspruch 18, der eine geradlinigie Reißfestigkeit im Bereich von 45.000 bis 70.000 psi, eine Knotenzugstärke im Bereich von 30.000 bis 50.000 psi und ein Youngsches Elastizitätsmodul im Bereich von 30.000 bis 180.000 psi hat.

21. Ein Triblock-Copolymer des Typs ABA, umfassend einen *p*-Dioxanon-A-Block und einen Segment-Copolymer-B-Block umfassend ε-Caprolacton und Trimethylencarbonat, wobei die inhärente Viskosität des Block-Copolymers im Bereich von 0,8 bis 4,0 dL/g ist, wie gemessen in einer 0,1 g/dL-Lösung aus Hexafluorisapropanol bei 25°C.

22. Das Triblock-Copolymer nach Anspruch 21, wobei der Gehalt an dem *p*-Dioxanon-A-Block im Bereich von 10 bis 90 mol% ist.

23. Das Triblock-Copolymer nach Anspruch 21, wobei der Gehalt an dem Segment-Copolymer-B-Block aus ε-Caprolacton und Trimethylencarbonat im Bereich von 10 bis 90 mol% ist.

24. Das Triblock-Copolymer nach Anspruch 21, wobei das Molverhältnis von ε-Caprolacton/Trimethylencarbonat in dem Segment-Copolymer-B-Block im Bereich von 5/95 bis 95/5 ist.

25. Das Triblock-Copolymer nach Anspruch 24, wobei das Molverhältnis von ε-Caprolacton/Trimethylencarbonat in dem Segment-Copolymer-B-Block im Bereich von 10/90 bis 90/10 ist.

26. Das Triblock-Copolymer nach Anspruch 21, wobei die inhärente Viskosität des Block-Copolymers im Bereich von 1,0 bis 3,5 dL/g ist, wie gemessen in einer 0,1 g/dL-Lösung aus Hexafluorisapropanol bei 25 °C.

27. Das Triblock-Copolymer nach Anspruch 21, welches einen Youngschen Elastizitätsmodul von 180.000 psi oder weniger hat.

28. Das Triblock-Copolymer nach Anspruch 21, wobei der Grad an Kristallinität des Block-Copolymers 10 % oder mehr ist.

29. Das Triblock-Copolymer nach Anspruch 28, wobei der Grad an Kristallinität des besagten Block-Copolymers 20 bis 27 % ist.

30. Ein Triblock-Copolymer des Typs ABA nach Anspruch 21, wobei der Gehalt an dem *p*-Dioxanon-A-Block im Bereich von 10 bis 90 mol% ist, das Molverhältnis von ε-Caprolacton/Trimethylencarbonat in dem Segment-Copolymer-B-Block im Bereich von 5/95 bis 95/5 ist.

31. Ein chirurgischer Gegenstand, umfassend ein Triblock-Copolymer des Typs ABA umfassend einen *p*-Dioxanon-A-Block und einen Segment-Copolymer-B-Block umfassend ε-Caprolacton und Trimethylencarbonat, wobei die inhärente Viskosität des Block-Copolymers im Bereich von 0,8 bis 4,0 dL/g ist, wie gemessen in einer 0,1 g/dL-Lösung aus Hexafluorisopropanol bei 25 °C.

32. Der chirurgische Gegenstand nach Anspruch 31, wobei der chirurgische Gegenstand ausgewählt ist aus der Gruppe bestehend aus chirurgischen Fäden, chirurgischen Schrauben, chirurgischen Klammern, chirurgischen Klemmen, Stiften, Anastomosenringen, chirurgischen Netzen und prothetischen Vorrichtungen.

33. Der chirurgische Gegenstand nach Anspruch 31, wobei besagter chirurgischer Faden an Nadeln befestigt ist.

34. Der chirurgische Gegenstand nach Anspruch 31, wobei die geradlinige Reißfestigkeit des chirurgischen Fadens im Bereich von 45.000 bis 70.000 psi ist.

35. Der chirurgische Gegenstand nach Anspruch 31, wobei die Knotenzugstärke des chirurgischen Fadens im Bereich von 30.000 bis 50.000 psi ist.

36. Der chirurgische Gegenstand nach Anspruch 31, wobei der Youngsche Elastizitätsmodul des chirurgischen Fadens im Bereich von 30.000 bis 180.000 psi ist.

37. Ein chirurgischer Gegenstand nach Anspruch 31, wobei der Gehalt an dem *p-*Dioxanon-A-Block im Bereich von 10 bis 90 mol% ist und das Molverhältnis von ε-Caprolacton/Trimethylencarbonat in dem Segment Copolymer-B-Block im Bereich von 5/95 bis 95/5 ist.

38. Der chirurgische Gegenstand nach Anspruch 37, wobei der chirurgische Gegenstand ausgewählt ist aus der Gruppe bestehend aus chirurgischen Fäden, chirurgischen Schrauben, chirurgischen Klemmen, chirurgischen Klammern, Stiften, Anastomosenringen, chirurgischen Netzen und prothetischen Vorrichtungen.

39. Der chirurgische Gegenstand nach Anspruch 37 hat eine geradlinige Reißfestigkeit im Bereich von 45.000 bis 70.000 psi, Knotenzugstärke im Bereich von 30.000 bis 50.000 psi und ein Youngsches Elastizitätsmodul im Bereich von 30.000 bis 180.000 psi.

40. Ein Verfahren zur Herstellung eines Block-Copolymers umfassend die Schritte:
(a) polymerisieren von ε-Caprolacton und Trimethylencarbonat zur Herstellung eines Segment-Copolymer-Blocks, wobei das Molverhältnis von ε-Caprolacton/Trimethylencarbonat in dem Segment-Copolymer im Bereich von 10/90 bis 90/10 ist; und
(b) umsetzen des Segment-Copolymer-Blocks mit *p*-Dioxanon-Monomeren, wobei der Gehalt an dem *p*-Dioxanon im Bereich von 5 bis 95 mol% ist zur Herstellung eines Block-Copolymers umfassend *p*-Dioxanon-Blöcke und Segment-Copolymer-Blöcke von ε-Caprolacton und Trimethylencarbonat.

41. Ein Block-Copolymer des Typs AB oder ABA umfassend *p*-Dioxanon-A-Blöcke und Segment-Copolymer-B-Blöcke von ε-Caprolacton und Trimethylencarbonat, hergestellt gemäß dem Verfahren nach Anspruch 40.

42. Ein Block-Copolymer nach Anspruch 1 oder 2 umfassend einen A-Block aus Polydioxanon, dargestellt durch Formel (1) und einen B-Block umfassend ein Segment-Copolymer aus ε-Caprolacton und Trimethylencarbonat, wobei der B-Block durch Formel (2) dargestellt wird, 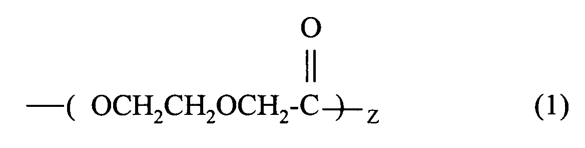 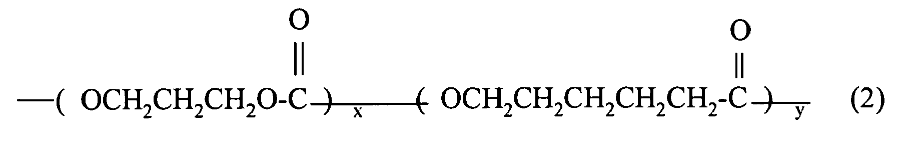 wobei x, y und z eine ganze Zahl zwischen 10 und 10⁴ darstellen und die inhärente Viskosität des Block-Copolymers im Bereich von 0,8 bis 4,0 dL/g ist, wie gemessen in einer 0,1 g/dL-Lösung von Hexafluorisopropanol bei 25 °C.

43. Das Block-Copolymer nach Anspruch 42, welches ein Block-Copolymer ist, enthaltend 5 bis 95 mol% der A-Blöcke und 5 bis 95 mol% der B-Blöcke.

44. Das Block-Copolymer nach Anspruch 43, welches ein Block-Copolymer ist, enthaltend 10 bis 90 mol% der A-Blöcke und 10 bis 90 mol% der B-Blöcke.

45. Das Block-Copolymer nach Anspruch 42, wobei der Grad an Kristallinität des Block-Copolymers 10 % oder mehr ist.

46. Das Block-Copolymer nach Anspruch 45, wobei der Grad an Kristallinität des Block-Copolymers 10 bis 27 % ist.

## Revendications

1. Copolymère à deux blocs de type AB comprenant un bloc A de *p*-dioxanone et un bloc B de copolymère segmenté de ε-caprolactone et de carbonate de triméthylène, la viscosité intrinsèque dudit copolymère à deux blocs de type AB se situant dans la fourchette de 0,8 à 4,0 dl/g telle que mesurée dans une solution à 0,1 g/dl d'hexafluoroisopropanol à 25°C.

2. Copolymère à deux blocs selon la revendication 1, dans lequel la teneur dudit bloc A de *p*-dioxanone se situe dans la fourchette de 5 à 95 % en mole.

3. Copolymère à deux blocs selon la revendication 2, dans lequel la teneur dudit bloc de *p*-dioxanone se situe dans la fourchette de 60 à 90 % en mole.

4. Copolymère à deux blocs selon la revendication 1, dans lequel la teneur dudit bloc B de copolymère segmenté de ε-caprolactone et de carbonate de triméthylène se situe dans la fourchette de 5 à 95 % en mole.

5. Copolymère à deux blocs selon la revendication 1, dans lequel le rapport molaire ε-caprolactone/carbonate de triméthylène dans ledit bloc B de copolymère segmenté se situe dans la fourchette de 5/95 à 95/5.

6. Copolymère à deux blocs selon la revendication 5, dans lequel le rapport molaire ε-caprolactone/carbonate de triméthylène dans ledit bloc B de copolymère segmenté se situe dans la fourchette de 10/90 à 90/10.

7. Copolymère à deux blocs selon la revendication 1, la viscosité intrinsèque dudit copolymère séquencé se situant dans la fourchette de 1,0 à 3,5 dl/g telle que mesurée dans une solution à 0,1 g/dl d'hexafluoroisopropanol à 25°C.

8. Copolymère à deux blocs selon la revendication 1 qui possède un module d'Young de 180 000 psi ou moins.

9. Copolymère à deux blocs selon la revendication 1, le degré de cristallinité dudit copolymère séquencé étant de 10 % ou plus.

10. Copolymère à deux blocs selon la revendication 9, le degré de cristallinité dudit copolymère séquencé étant de 20 à 27 %.

11. Copolymère à deux blocs de type AB selon la revendication 1, dans lequel la teneur dudit bloc A de *p*-dioxanone se situe dans la fourchette de 5 à 95 % en mole et le rapport molaire ε-caprolactone/carbonate de triméthylène dans ledit bloc B de copolymère segmenté se situe dans la fourchette de 5/95 à 95/5.

12. Article chirurgical comprenant un copolymère à deux blocs de type AB comprenant un bloc A de *p*-dioxanone et un bloc B de copolymère segmenté de ε-caprolactone et de carbonate de triméthylène, dans lequel la viscosité intrinsèque dudit copolymère séquencé de type AB se situe dans la fourchette de 0,8 à 4,0 dl/g telle que mesurée dans une solution à 0,1 g/dl d'hexafluoroisopropanol à 25°C.

13. Article chirurgical selon la revendication 12, l'article chirurgical étant un élément choisi dans le groupe constitué par les sutures chirurgicales, les vis chirurgicales, les agrafes chirurgicales, les attaches chirurgicales, les broches, les anneaux d'anastomose, les treillis chirurgicaux et les dispositifs prothétiques.

14. Article chirurgical selon la revendication 13, dans lequel ladite suture chirurgicale est attachée à des aiguilles.

15. Article chirurgical selon la revendication 13, dans lequel la résistance à la traction rectiligne de ladite suture chirurgicale se situe dans la fourchette de 45 000 à 70 000 psi.

16. Article chirurgical selon la revendication 13, dans lequel la résistance à la traction d'un noeud de ladite suture chirurgicale se situe dans la fourchette de 30 000 50 000 psi.

17. Article chirurgical selon la revendication 13, dans lequel le module d'Young de ladite suture chirurgicale se situe dans la fourchette de 30 000 à 180 000 psi.

18. Article chirurgical selon la revendication 12, dans lequel la teneur dudit bloc A de *p*-dioxanone se situe dans la fourchette de 5 à 95 % en mole et le rapport molaire ε-caprolactone/carbonate de triméthylène dans ledit bloc B de copolymère segmenté se situe dans la fourchette de 5/95 à 95/5.

19. Article chirurgical selon la revendication 18, l'article chirurgical étant un élément choisi dans le groupe constitué par les sutures chirurgicales, les vis chirurgicales, les agrafes chirurgicales, les attaches chirurgicales, les broches, les anneaux d'anastomose, les treillis chirurgicaux et les dispositifs prothétiques.

20. Article chirurgical selon la revendication 18 qui possède une résistance à la traction rectiligne dans la fourchette de 45 000 à 70 000 psi, une résistance à la traction d'un noeud dans la fourchette de 30 000 à 50 000 psi, et un module d'Young dans la fourchette de 30 000 à 180 000 psi.

21. Copolymère à trois blocs de type ABA comprenant un bloc A de *p*-dioxanone et un bloc B de copolymère segmenté de ε-caprolactone et de carbonate de triméthylène, la viscosité intrinsèque dudit copolymère séquencé se situant dans la fourchette de 0,8 à 4,0 dl/g telle que mesurée dans une solution à 0,1 g/dl d'hexafluoroisopropanol à 25°C.

22. Copolymère à trois blocs selon la revendication 21, dans lequel la teneur dudit bloc A de *p*-dioxanone se situe dans la fourchette de 10 à 90 % en mole.

23. Copolymère à trois blocs selon la revendication 21, dans lequel la teneur dudit bloc B de copolymère segmenté de ε-caprolactone et de carbonate de triméthylène se situe dans la fourchette de 10 à 90 % en mole.

24. Copolymère à trois blocs selon la revendication 21, dans lequel le rapport molaire ε-caprolactone/carbonate de triméthylène dans ledit bloc B de copolymère segmenté se situe dans la fourchette de 5/95 à 95/5.

25. Copolymère à trois blocs selon la revendication 24, dans lequel le rapport molaire ε-caprolactone/carbonate de triméthylène dans ledit bloc B de copolymère segmenté se situe dans la fourchette de 10/90 à 90/10.

26. Copolymère à trois blocs selon la revendication 21, la viscosité intrinsèque dudit copolymère séquencé se situant dans la fourchette de 1,0 à 3,5 dl/g telle que mesurée dans une solution à 0,1 g/dl d'hexafluoroisopropanol à 25°C.

27. Copolymère à trois blocs selon la revendication 21 qui possède un module d'Young de 180 000 psi ou moins.

28. Copolymère à trois blocs selon la revendication 21, le degré de cristallinité dudit copolymère séquencé étant de 10 % ou plus.

29. Copolymère à trois blocs selon la revendication 28, le degré de cristallinité dudit copolymère séquencé étant de 20 à 27 %.

30. Copolymère à trois blocs de type ABA selon la revendication 21, dans lequel la teneur dudit bloc A de *p*-dioxanone se situe dans la fourchette de 10 à 90 % en mole et le rapport molaire ε-caprolactone/carbonate de triméthylène dans ledit bloc B de copolymère segmenté se situe dans la fourchette de 5/95 à 95/5.

31. Article chirurgical comprenant un copolymère à trois blocs de type ABA comprenant un bloc A de *p*-dioxanone et un bloc B de copolymère segmenté de ε-caprolactone et de carbonate de triméthylène, dans lequel la viscosité intrinsèque dudit copolymère séquencé se situe dans la fourchette de 0,8 à 4,0 dl/g telle que mesurée dans une solution à 0,1 g/dl d'hexafluoroisopropanol à 25°C.

32. Article chirurgical selon la revendication 31, l'article chirurgical étant un élément choisi dans le groupe constitué par les sutures chirurgicales, les vis chirurgicales, les agrafes chirurgicales, les attaches chirurgicales, les broches, les anneaux d'anastomose, les treillis chirurgicaux et les dispositifs prothétiques.

33. Article chirurgical selon la revendication 31, dans lequel ladite suture chirurgicale est attachée à des aiguilles.

34. Article chirurgical selon la revendication 31, dans lequel la résistance à la traction rectiligne de ladite suture chirurgicale se situe dans la fourchette de 45 000 à 70 000 psi.

35. Article chirurgical selon la revendication 31, dans lequel la résistance à la traction d'un noeud de ladite suture chirurgicale se situe dans la fourchette de 30 000 50 000 psi.

36. Article chirurgical selon la revendication 31, dans lequel le module d'Young de ladite suture chirurgicale se situe dans la fourchette de 30 000 à 180 000 psi.

37. Article chirurgical selon la revendication 31, dans lequel la teneur dudit bloc A de *p*-dioxanone se situe dans la fourchette de 10 à 90 % en mole et le rapport molaire ε-caprolactone/carbonate de triméthylène dans ledit bloc B de copolymère segmenté se situe dans la fourchette de 5/95 à 95/5.

38. Article chirurgical selon la revendication 37, l'article chirurgical étant un élément choisi dans le groupe constitué par les sutures chirurgicales, les vis chirurgicales, les agrafes chirurgicales, les attaches chirurgicales, les broches, les anneaux d'anastomose, les treillis chirurgicaux et les dispositifs prothétiques.

39. Article chirurgical selon la revendication 37 qui possède une résistance à la traction rectiligne dans la fourchette de 45 000 à 70 000 psi, une résistance à la traction d'un noeud dans la fourchette de 30 000 à 50 000 psi, et un module d'Young dans la fourchette de 30 000 à 180 000 psi.

40. Procédé de préparation d'un copolymère séquencé comprenant les états de :
(a) polymérisation de ε-caprolactone et de carbonate de triméthylène pour produire un bloc de copolymère segmenté dans lequel le rapport molaire ε-caprolactone/carbonate de triméthylène dans ledit copolymère segmenté se situe dans la fourchette de 10/90 à 90/10 ; et
(b) réaction du bloc de copolymère segmenté avec des monomères de *p*-dioxanone dans lesquels la teneur de ladite *p*-dioxanone se situe dans la fourchette de 5 à 95 % en mole pour produire un copolymère séquencé comprenant des blocs de *p*-dioxanone et des blocs de copolymère segmenté de ε-caprolactone et de carbonate de triméthylène.

41. Copolymère séquencé de type AB ou ABA comprenant des blocs de *p*-dioxanone et des blocs de copolymère segmenté de ε-caprolactone et de carbonate de triméthylène préparé par le procédé de la revendication 40.

42. Copolymère séquencé selon la revendication 1 ou 2 comprenant un bloc A de polydioxanone représenté par la formule (1) et un bloc B comprenant un copolymère segmenté de ε-caprolactone et de carbonate de triméthylène, ledit bloc B étant représenté par la formule (2) : 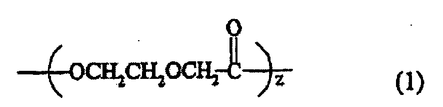 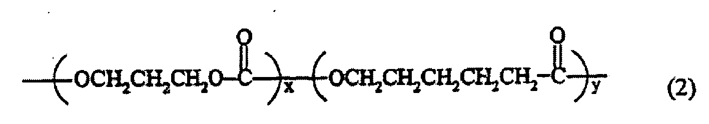 dans lequel x, y et z représentent un entier entre 10 et 10⁴, et la viscosité intrinsèque dudit copolymère séquencé se situe dans la fourchette de 0,8 à 4,0 dl/g telle que mesurée dans une solution à 0,1 g/dl d'hexafluoroisopropanol à 25°C.

43. Copolymère séquencé selon la revendication 42 qui est un copolymère séquencé contenant 5 à 95 % en mole desdits blocs A et 5 à 95 % en mole desdits blocs B.

44. Copolymère séquencé selon la revendication 43 qui est un copolymère séquencé contenant 10 à 90 % en mole desdits blocs A et 10 à 90 % en mole desdits blocs B.

45. Copolymère séquencé selon la revendication 42, le degré de cristallinité dudit copolymère séquencé étant de 10 % ou plus.

46. Copolymère séquencé selon la revendication 45, le degré de cristallinité dudit copolymère séquencé étant de 10 à 27 %.
